# EUROPÄISCHE PATENTSCHRIFT

(11) **EP 3 491 145 B1**
(45) Veröffentlichungstag und Bekanntmachung des Hinweises auf die Patenterteilung: **20.04.2022**
(21) Anmeldenummer: 17748696.6
(22) Anmeldetag: 25.07.2017
(51) Int. Cl.: C12Q 1/6881

(54) **MARKER UND VERFAHREN ZUR BEURTEILUNG DER ZUSAMMENSETZUNG ODER REINHEIT EINER ZELLKULTUR**
MARKER AND METHOD FOR ASSESSING THE COMPOSITION OR PURITY OF A CELL CULTURE
MARQUEUR ET PROCÉDÉ D'ÉVALUATION DE LA COMPOSITION OU DE LA PURETÉ D'UNE CULTURE CELLULAIRE

(30) Priorität: 26.07.2016 DE 102016213684
(43) Veröffentlichungstag der Anmeldung: 05.06.2019
(73) Patentinhaber: TETEC Tissue Engineering Technologies AG, 72770 Reutlingen (DE)
(72) Erfinder: BENZ, Karin, 73037 Göppingen (DE); GAISSMAIER, Christoph, 72127 Kusterdingen (DE)
(74) Vertreter: Patentanwälte Ruff, Wilhelm, Beier, Dauster & Partner mbB
(86) Internationale Anmeldenummer: PCT/EP2017/068781
(87) Internationale Veröffentlichungsnummer: WO 2018/019839

(56) Entgegenhaltungen:
- WO-A1-2011/148025
- STEPHEN RAPKO ET AL: "Identification of the Chondrocyte Lineage Using Microfibril-Associated Glycoprotein-2, A Novel Marker That Distinguishes Chondrocytes from Synovial Cells", TISSUE ENGINEERING. PART C, METHODS DEC 2008, Bd. 16, Nr. 6, 1. Dezember 2010 (2010-12-01), Seiten 1367-1375, XP055411817, US ISSN: 1937-3384, DOI: 10.1089/ten.tec.2009.0772
- Iowa Research Online ET AL: "University of Iowa Discriminating Chondrogenic Progenitor Cells (CPCs) as a Distinct Cell Type, Apart from Normal Chondrocytes", , 1. August 2013 (2013-08-01), XP055411887, Gefunden im Internet: URL:http://ir.uiowa.edu/cgi/viewcontent.cg i?article=4890&context=etd [gefunden am 2017-10-02]
- HISSNAUER T N ET AL: "Identification of molecular markers for articular cartilage", OSTEOARTHRITIS AND CARTILAGE, BAILLIERE TINDALL, LONDON, GB, Bd. 18, Nr. 12, 1. Dezember 2010 (2010-12-01), Seiten 1630-1638, XP027523837, ISSN: 1063-4584 [gefunden am 2010-10-13]

## Beschreibung

### ANWENDUNGSGEBIET UND STAND DER TECHNIK

Die Erfindung betrifft Marker zur Beurteilung der Zusammensetzung oder Reinheit einer Zellkultur, ein Verfahren zur Beurteilung der Zusammensetzung oder Reinheit einer Zellkultur, die Verwendung einer Zellkultur zur Herstellung eines Arzneimittels für neuartige Therapien sowie die Verwendung eines Kits zur Durchführung des vorstehend genannten Verfahrens.

An die Qualität von zellbasierten Arzneimitteln, insbesondere zellbasierten Tissue-Engineering-Produkten, werden zunehmend hohe Anforderungen gestellt. Dies gilt insbesondere im Hinblick auf die Reinheit sowie Identität von zur Herstellung von zellbasierten Arzneimitteln verwendeten Zellen.

Dies stellt insbesondere für Knorpelzellkulturen ein großes Problem dar. Derartige Kulturen werden häufig unter Verwendung von autologen Knorpelzellen angesetzt und kommen nach einer bestimmten Kultivierungszeit im Rahmen einer Matrix-assoziierten autologen Knorpelzelltransplantation zum Einsatz.

Die besondere Problematik besteht darin, dass sich die verschiedenen Zelltypen mesenchymalen Ursprungs, wie beispielsweise Osteoblasten, Synovialzellen sowie mesenchymale Stammzellen, nicht sehr unterscheiden und daher mittels herkömmlicher Marker praktisch nicht zu unterscheiden sind.

Als kontaminierende Zelltypen können bei Knorpelzellkulturen grundsätzlich Endothelzellen, Osteoblasten sowie Synovialzellen als bedeutsam angesehen werden. Besonders kritisch sind jedoch Synovialzellen, da sie sich - im Gegensatz zu Endothelzellen und Osteoblasten - unter üblichen Kulturbedingungen häufig ähnlich wie Knorpelzellen verhalten.

Verfahren zur Beurteilung von Zellen und Zellkulturen sind bereits aus der EP 2 132 562 B9 bekannt.

Stephen Rapko et al. (Identification of the Chondrocyte Lineage Using Microfibril-Associated Glycoprotein-2, A Novel Marker That Distinguishes Chrondocytes from Synovial Cells, TISSUE ENGINEERING: Part C, V. 16, Nr. 6, 2010, p. 1367-1375) offenbart CRTL1 sowie MAGP2 als Marker zur Unterscheidung zwischen Knorpelzellkulturen und Synovialzellkulturen. Gegenstand der WO 2011/148025 A1 ist die Verwendung des Biomarkers SUSD2 zur Identifizierung von Knorpelzellen gegenüber mesenchymalen Zellen in einer Zellkultur.

Gleichwohl besteht weiterhin ein Bedarf an alternativen oder verbesserten Möglichkeiten zur Durchführung von Reinheitskontrollen bei Zellkulturen sowie zur Identitätsbestimmung von Zellen.

### AUFGABE UND LÖSUNG

Die Erfindung stellt sich daher die Aufgabe, Marker bereitzustellen, welche eine Reinheitskontrolle von Knorpelzellkulturen ermöglichen.

Darüber hinaus stellt sich die Erfindung die Aufgabe, die Verwendung einer Zellkultur zur Herstellung eines Arzneimittels für neuartige Therapien bereitzustellen.

Diese Aufgaben werden erfindungsgemäß gelöst durch die Verwendung eines Markers gemäß dem unabhängigen Anspruch 1, durch ein Verfahren gemäß dem Anspruch 8 sowie durch die Verwendung eines Kits gemäß Anspruch 9. Bevorzugte Ausführungsformen sind in den abhängigen Ansprüchen definiert.

Gemäß einem ersten Aspekt betrifft die Erfindung die Verwendung eines Markers zur Beurteilung, insbesondere zur Bestimmung, der Zusammensetzung oder Reinheit einer Zellkultur.

Der Marker zeichnet sich besonders dadurch aus, dass es sich um wenigstens einen Marker handelt, welcher aus der Gruppe bestehend aus MMP1, ACE, POSTN, SYNPO, SULF1, ARHGEF28, IGF2BP1, BAIAP2L1, LIMCH1, SCIN, DCLK1, PPL, CRABP2, NES, XPNPEP2 und SLIT3 ausgewählt ist, wobei das Expressionsniveau des wenigstens einen Markers bestimmt wird.

Der Ausdruck "wenigstens ein Marker" kann im Sinne der vorliegenden Erfindung einen der oben genannten Marker oder eine Kombination von zwei oder mehr Markern, d.h. eine Kombination aus zwei, drei, vier, fünf, sechs, sieben, acht, neun, zehn, elf, zwölf, dreizehn, vierzehn, fünfzehn, oder sechzehn, der oben genannten Marker oder eine Kombination von allen der oben genannten Marker bedeuten. Insbesondere kann der Ausdruck "wenigstens ein Marker" im Sinne der vorliegenden Erfindung ein Verhältnis aus zwei der oben genannten Marker bedeuten.

Weiterhin kann der Ausdruck "Marker" im Sinne der vorliegenden Erfindung allgemein ein Genprodukt, d.h. das Ergebnis der Expression, wie insbesondere die messenger RNA (mRNA), auch als Boten-RNA bezeichnet, oder das Protein, eines oder mehrerer der oben genannten Marker bedeuten.

Unter dem Ausdruck "Expressionsniveau" soll im Sinne der vorliegenden Erfindung ein Expressionsniveau auf RNA-Basis, insbesondere messenger RNA-Basis (mRNA-Basis), und/oder ein Expressionsniveau auf Protein-Basis verstanden werden.

Der in der vorliegenden Erfindung im Zusammenhang mit dem Ausdruck "Expressionsniveau" verwendete Ausdruck "bestimmen" soll im Sinne der vorliegenden Erfindung jedes biochemische und/oder biotechnologische Verfahren umfassen, mittels dessen das Expressionsniveau des wenigstens einen Markers, insbesondere auf RNA- oder Protein-Basis, erfasst, insbesondere quantitativ erfasst, werden kann.

Den Erfindern ist es im Rahmen einer auf massenspektroskopischen Untersuchungen basierenden Proteom-Analyse mit nachgeschalteter bioinformatischer Auswertung gelungen, Knorpelzellmarker, d.h. Chondrozytenmarker, sowie Synovialzellmarker, d.h. Synoviozytenmarker, zu identifizieren, welche dazu geeignet sind, eine Reinheitskontrolle von Knorpelzellkulturen, und/oder einen Nachweis der Identität von Knorpelzellen, d.h. Chondrozyten, und/oder Synovialzellen, d.h. Synoviozyten bzw. Synovialfibroblasten, zu ermöglichen. Die im Rahmen der vorliegenden Erfindung identifizierten Marker erlauben insbesondere den Nachweis einer Synovialzellverunreinigung in Knorpelzellkulturen und/oder den Nachweis der Anwesenheit von Knorpelzellen in einer Synovialzellkultur und/oder die Diskriminierung zwischen Knorpelzellen und Synovialzellen.

Die erfindungsgemäß vorgeschlagenen Marker können daher auch als Knorpellzell- bzw. Chondrozytenmarker, d.h. als Reinheits- bzw. Qualitätsmarker und/oder Identitätsmarker für Knorpelzellen bzw. Chondrozyten, und/oder als Synovialzell- bzw. Synoviozytenmarker, d.h. als Reinheits- bzw. Qualitätsmarker und/oder Identitätsmarker für Synovialzellen bzw. Synoviozyten, bezeichnet werden.

In einer bevorzugten Ausführungsform wird das Expressionsniveau des wenigstens einen Markers in einer aus der Zellkultur gewonnenen Zellprobe bestimmt.

Unter dem Ausdruck "Zellprobe" soll im Sinne der vorliegenden Erfindung eine zellhaltige oder eine aus Zellen bestehende Probe verstanden werden, welche der Zellkultur entnommen wird.

POSTN wird als Protein auch als "Periostin" oder "Osteoblast-spezifischer Faktor OSF-2" bezeichnet. Periostin ist ein Ligand für alpha-V/beta-3 sowie alpha-V/beta-5 Integrine zur Unterstützung der Adhäsion und Migration von Epithelzellen.

Als Protein wird SYNPO auch als "Synaptopodin" bezeichnet. Synaptopodin ist ein cytoplasmatisches aktinassoziiertes Protein mit einer Molekülmasse von etwa 73 kDa. Synaptopodin kommt sowohl in den Podocyten von Nieren als auch im Großhirn vor.

Als Protein wird MMP1 auch als "Matrix-Metalloproteinase-1", "Interstitiale Collagenase" oder "Fibroblast Kollagenase" bezeichnet.

Als Protein wird SULF1 auch als "Sulfatase 1" bezeichnet. Sulfatase 1 ist ein Enzym, welches selektiv 6-O-Sulfatgruppen von Heparansulfat entfernt.

Als Protein wird ARHGEF28 auch als "Rho Guanine Nucleotide Exchange Factor 28" bezeichnet.

Als Protein wird IGF2BP1 auch als "Insulin-Like Growth Factor 2 mRNA Binding Protein 1" bezeichnet.

Als Protein wird BAIAP2L1 auch als "Brain-specific angiogenesis inhibitor 1-associated protein 2-like protein 1 (BAI1-associated protein 2-like protein 1)" bezeichnet.

Als Protein wird LIMCH1 auch als "LIM and Calponin Homology Domains 1" bezeichnet.

Als Protein wird SCIN auch als "Scinderin" oder "Adseverin" bezeichnet.

Als Protein wird DCLK1 auch als "doublecortin like kinase-1" bezeichnet.

Als Protein wird PPL auch als "Periplakin" bezeichnet.

Als Protein wird CRABP2 auch als "cellular retinoic acid-binding protein 2" bezeichnet.

Als Protein wird NES auch als "Nestin" bezeichnet.

Als Protein wird XPNPEP2 auch als "X-Prolyl Aminopeptidase" bezeichnet.

Als Protein wird SLIT3 auch als "Slit Guidance Ligand 3" bezeichnet.

Bei dem wenigstens einen Marker handelt es sich um ACE. Als Protein wird ACE auch als "Angiotensin converting enzyme (Angiotensin-konvertierendes Enzym)" bezeichnet.

Bei dem wenigstens einen Marker handelt es sich um eine Kombination von ACE und wenigstens einem weiteren Marker, ausgewählt aus der Gruppe bestehend aus MMP1, POSTN, SYNPO, SULF1, ARHGEF28, IGF2BP1, BAIAP2L1, LIMCH1, SCIN, DCLK1, PPL, CRABP2, NES, XPNPEP2 und SLIT3. Besonders bevorzugt handelt es sich bei dem wenigstens einen Marker um eine Kombination von ACE und MMP1.

Weiterhin kann es sich bei dem wenigstens einen Marker insbesondere um eine Kombination von ACE und wenigstens einem weiteren Marker, ausgewählt aus der Gruppe bestehend aus DCLK1, PPL, CRABP2, NES, XPNPEP2 und SLIT3, handeln.

Bei der Zellkultur handelt es sich um eine Knorpelzellkultur bzw. Chondrozytenkultur.

Unter dem Ausdruck "Knorpelzellkultur" bzw. "Chondrozytenkultur" soll im Sinne der vorliegenden Erfindung eine Kultur verstanden werden, welche ausgehend von Knorpelgewebe, bevorzugt ausgehend von einem Knorpelbiopsat, insbesondere Knorpel-Knochen-Stanzbiopsat, mittels enzymatischem Verdau und anschließender Kultivierung der nach dem Verdau erhaltenen Zellen hergestellt bzw. herstellbar ist.

Der Ausdruck "Knorpelgewebe" kann im Sinne der vorliegenden Erfindung gesundes Knorpelgewebe, erkranktes oder krankhaft verändertes Knorpelgewebe oder ein Knorpelgewebe-Regenerat, das heißt ein vorzugsweise infolge medizinischer Behandlung, insbesondere einer autologen Chondrozyten-Transplantation (ACT) oder Matrix-assoziierten autologen Chondrozyten-Transplantation (MACT), neu entstandenes Knorpelgewebe, bedeuten.

Der Ausdruck "Knorpelbiopsat" kann im Sinne der vorliegenden Erfindung ein Biopsat bedeuten, welches gesundes Knorpelgewebe, erkranktes oder krankhaft verändertes Knorpelgewebe oder ein Knorpelgewebe-Regenerat enthält oder aus einem der vorgenannten Gewebe besteht. Der Ausdruck "Knorpel-Knochen-Stanzbiopsat" soll im Sinne der vorliegenden Erfindung ein Biopsat bedeuten, welches gesundes Knochen- und Knorpelgewebe, erkranktes Knochen- und Knorpelgewebe oder krankhaft verändertes Knochen- und Knorpelgewebe oder ein Knochen- und Knorpelgewebe-Regenerat enthält oder aus einem der vorgenannten Gewebe besteht.

Unter dem Ausdruck "Synovialzellkultur" bzw. "Synoviozytenkultur" soll im Sinne der vorliegenden Erfindung eine Kultur verstanden werden, welche ausgehend von Synovialgewebe mittels enzymatischem Verdau und anschließender Kultivierung der nach dem Verdau erhaltenen Zellen hergestellt bzw. herstellbar ist.

Die Zellkultur weist in einer weiteren Ausführungsform Zellen auf, welche aus einem Knorpelbiopsat stammen. Insbesondere kann die Zellkultur Zellen aufweisen, welche aus einem Knorpel-Knochen-Stanzbiopsat, insbesondere einem Knorpelgewebefragment davon, stammen.

Bei dem Biopsat, insbesondere Knorpelbiopsat oder Knorpel-Knochen-Stanzbiopsat, handelt es sich vorzugsweise um ein Biopsat aus einem Gelenk, insbesondere Kniegelenk.

Unter dem Ausdruck "reine Synovialzellkultur" soll im Sinne der vorliegenden Erfindung eine Synovialzellkultur verstanden werden, welche Zellen ausschließlich in Form von Synovialzellen bzw. Synoviozyten aufweist.

Unter dem Ausdruck "reine Knorpelzellkultur" soll im Sinne der vorliegenden Erfindung eine Knorpelzellkultur verstanden werden, welche Zellen ausschließlich in Form von Knorpelzellen bzw. Chondrozyten aufweist.

In einer weiteren Ausführungsform handelt es sich bei dem wenigstens einen Marker um das Verhältnis von ACE zu MMP1 (ACE/MMP1 Verhältnis) oder um das Verhältnis von MMP1 zu ACE (MMP1/ACE Verhältnis). Mit anderen Worten handelt es sich bei dem Marker zur Beurteilung der Zusammensetzung oder Reinheit einer Zellkultur gemäß einer weiteren Ausführungsform um das Verhältnis von ACE zu MMP1 oder um das Verhältnis von MMP1 zu ACE. Bevorzugt wird zur Beurteilung der Zusammensetzung oder Reinheit der Zellkultur das Verhältnis von ACE zu MMP1 gebildet, das heißt als Marker zur Beurteilung der Zusammensetzung oder Reinheit einer Zellkultur das ACE/MMP1 Verhältnis verwendet. Vorzugsweise zeigt ein Wert des ACE/MMP1 Verhältnisses oberhalb eines definierten Schwellenwertes an, dass die Zellkultur Synovialzellen (Synoviozyten) aufweist. Bevorzugt ist der Schwellenwert gleich oder kleiner als der Wert des Verhältnisses von ACE zu MMP1 in einer reinen Synovialzellkultur.

Das Expressionsniveau des wenigstens einen Markers wird in einer weiteren Ausführungsform auf RNA-Basis, vorzugsweise messenger-RNA-Basis (mRNA-Basis), bestimmt.

In einer weiteren Ausführungsform wird das Expressionsniveau des wenigstens einen Markers auf Protein-Basis bestimmt.

In einer weiteren Ausführungsform wird das Expressionsniveau des wenigstens einen Markers mittels Polymerase-Kettenreaktion wie Reverse Transkriptase-Polymerase-Kettenreaktion oder quantitative Echtzeit-Polymerase-Kettenreaktion, eines Elektrophorese-Assays, eines Gelelektrophorese-Assays wie Southern Blot, Northern Blot oder Western Blot, Protein-Chips, Antikörper, eines Immunassays wie ELISAs, eines LUMINEX-Immunassays oder ELISpot Assays, eines Immunfluoreszenz-Assays, eines immunhistochemischen Assays, eines radioimmunologischen Assays, Proteom-Analyse, eines Chromatographie-basierten Assays wie HPLC oder Gelchromatographie, eines massenspektroskopischen Assays oder eines Durchflusszytometrie-Assays bestimmt.

Gemäß einem zweiten Aspekt betrifft die Erfindung ein Verfahren zur Beurteilung, insbesondere zur Bestimmung, der Zusammensetzung oder Reinheit einer Zellkultur.

Das Verfahren weist die folgenden Schritte auf:
a) Gewinnen einer Zellprobe aus einer Zellkultur,
b) Bestimmen des Expressionsniveaus wenigstens eines Markers in der Zellprobe und
c) Bestimmen der Zusammensetzung oder Reinheit der Zellkultur basierend auf dem Expressionsniveau des wenigstens einen Markers.

Das Verfahren zeichnet sich besonders dadurch aus, dass der wenigstens eine Marker ACE oder eine Kombination von ACE und wenigstens einem weiteren Marker, ausgewählt aus der Gruppe bestehend aus MMP1, POSTN, SYNPO, SULF1, ARHGEF28, IGF2BP1, BAIAP2L1, LIMCH1, SCIN, DCLK1, PPL, CRABP2, NES, XPNPEP2 und SLIT3 ist.

Die Zellkultur weist in einer weiteren Ausführungsform Zellen auf, welche aus einem Knorpelbiopsat stammen. Insbesondere kann die Zellkultur Zellen aufweisen, welche aus einem Knorpel-Knochen-Stanzbiopsat, insbesondere einem Knorpelgewebefragment davon, stammen.

Bei dem Biopsat, insbesondere Knorpelbiopsat oder Knorpel-Knochen-Stanzbiopsat, handelt es sich vorzugsweise um ein Biopsat aus einem Gelenk, insbesondere Kniegelenk.

In einer weiteren Ausführungsform handelt es sich bei dem wenigstens einen Marker um das Verhältnis von ACE zu MMP1 (ACE/MMP1 Verhältnis) oder um das Verhältnis von MMP1 zu ACE (MMP1/ACE Verhältnis). Bevorzugt handelt es sich bei dem wenigstens einen Marker um das Verhältnis von ACE zu MMP1. Vorzugsweise zeigt ein Wert des Verhältnisses von ACE zu MMP1 oberhalb eines definierten Schwellenwertes an, dass die Zellkultur Synovialzellen (Synoviozyten) aufweist. Bevorzugt ist der Schwellenwert gleich oder kleiner als der Wert des Verhältnisses von ACE zu MMP1 in einer reinen Synovialzellkultur.

Bezüglich weiterer Merkmale und Vorteile des Verfahrens, insbesondere des wenigstens einen Markers, wird zur Vermeidung von Wiederholungen vollständig auf die im Rahmen des ersten Erfindungsaspekts gemachten Ausführungen Bezug genommen. Die dort beschriebenen Ausführungsformen gelten (sinngemäß) auch für das Verfahren gemäß zweitem Erfindungsaspekt.

Gemäß einem dritten Aspekt betrifft die Erfindung die Verwendung eines Kits zur Durchführung eines Verfahrens gemäß zweitem Erfindungsaspekt.

Das Kit weist wenigstens eine Komponente zum Nachweis wenigstens eines Markers gemäß erstem Erfindungsaspekt auf.

Bei der Komponente kann es sich insbesondere um einen Antikörper, wie beispielsweise Abfang- und/oder Detektionsantikörper, Farbstoff wie Fluoreszenzfarbstoff, Beads, Pufferlösung, Nährlösung, Waschlösung, Primer (Oligonukleotide, welche als Startpunkt für DNAreplizierende Enzyme wie die DNA-Polymerase dienen) oder um eine Mischung/Kombination von wenigstens zwei der genannten Komponenten handeln.

Bezüglich weiterer Merkmale und Vorteile des Kits, insbesondere des wenigstens einen Markers, wird zur Vermeidung von Wiederholungen vollständig auf die im Rahmen der bisherigen Beschreibung gemachten Ausführungen Bezug genommen. Die dort beschriebenen Ausführungsformen gelten (sinngemäß) auch für das Kit gemäß drittem Erfindungsaspekt.

Weitere Merkmale und Vorteile der Erfindung ergeben sich aus der nachfolgenden Beschreibung von bevorzugten Ausführungsformen in Form von Ausführungsbeispielen. Hierbei können einzelne Merkmale jeweils für sich alleine oder in Kombination miteinander verwirklicht sein. Die nachstehend beschriebenen Ausführungsbeispiele dienen lediglich der weiteren Erläuterung der Erfindung, ohne diese hierauf zu beschränken.

### FIGURENKURZBESCHREIBUNG

In den Figuren ist Folgendes gezeigt:
- Figur 1:: Scatterplot-Vergleich von log10-transformierten Synoviozyten/Chondrozyten LQF-Intensitäten,
- Figur 2:: Vulkanplot-Darstellung zur Visualisierung der Mittelwerte von log10-transformierten Synoviozyten/Chondrozyten LFQ-Intensitäten(mean LFQ ratios),,
- Figur 3:: Vulkan-Plot zur Visualisierung der Unterschiede im PeptideCount zwischen Synoviozyten und Chondrozyten und den zugehörigen Unterschieden im q-Wert für alle Proteine, die nicht im MeanRank-Test verwendet wurden,
- Figur 4:: mRNA-Expression des Synoviozyten-Markers ACE in Chondrozyten und Synoviozyten,
- Figur 5:: mRNA-Expression des Chondrozyten-Markers MMP1 in Chondrozyten und Synoviozyten,
- Figur 6:: Verhältnis der mRNA-Expression des Synoviozyten-Markers ACE und des Chondrozyten-Markers MMP1 in Chondrozyten und Synoviozyten,
- Figur 7:: Verhältnis der mRNA-Expression von ACE/MMP1 in Zellmischungen,
- Fig. 8:: mRNA-Expression von ACE in Zellmischungen und
- Fig. 9:: mRNA-Expression von MMP1 in Zellmischungen.

### AUSFÜHRLICHE FIGURENBESCHREIBUNG

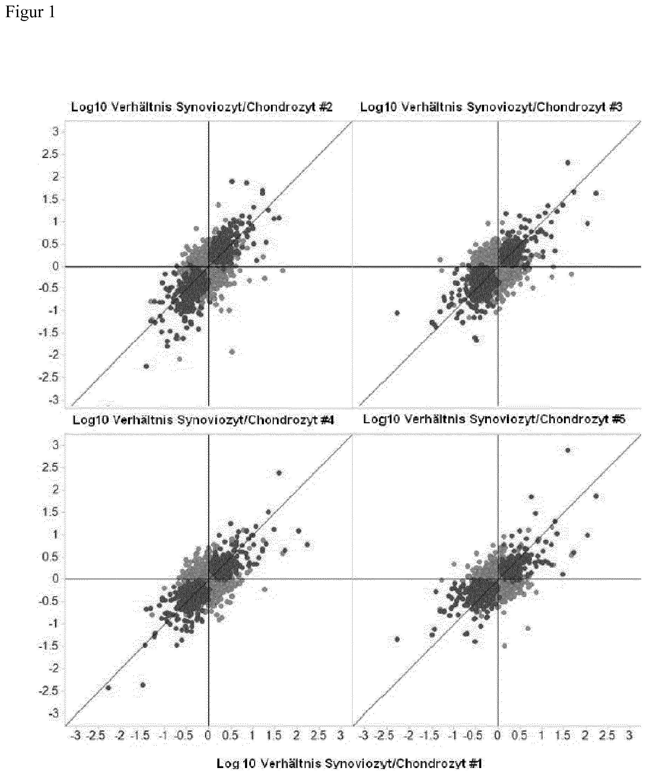

Fig. 1 zeigt einen Scatterplot-Vergleich der log10-transformierten Synoviozyten/Chondrozyten LQF-Intensitäten von Donor 1 (x-Achse) zu den anderen vier Donoren. Die Ursprungsgerade mit der Steigung 1 entspricht einer 100%igen Korrelation zwischen den Wiederholungsexperimenten. Proteine, die im Mean Rank-Test als in einem Zelltyp höher exprimiert eingestuft wurden, sind dunkler dargestellt.

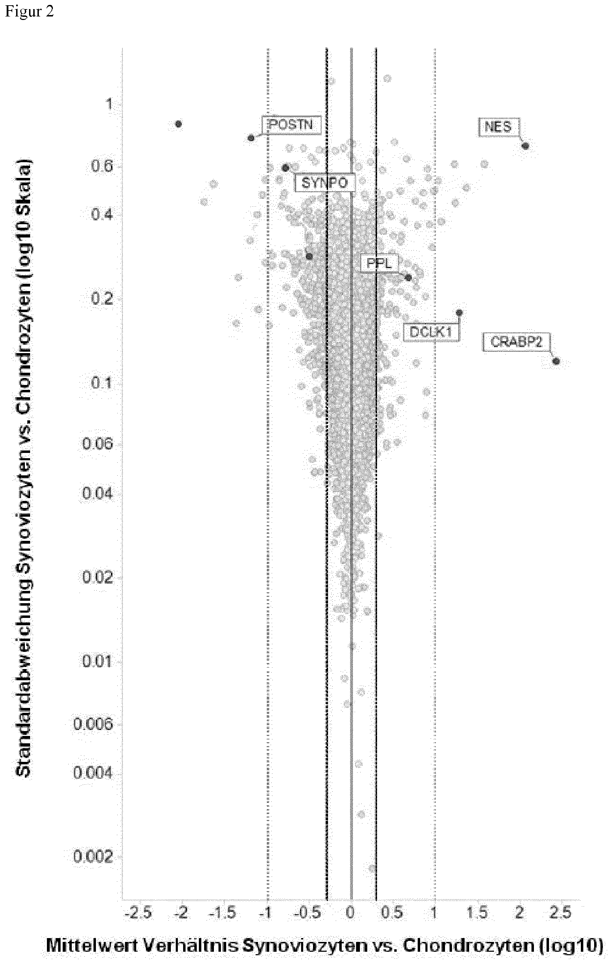

Fig. 2 zeigt eine Vulkanplot-Darstellung zur Visualisierung der Mittelwerte von log10-transformierten Synoviozyten/Chondrozyten LFQ-Intensitäten (Mean LFQ Ratios) gegenüber den Standardabweichungen der Verhältnisse aller Proteine, die in mindestens 3 von 5 Wiederholungsexperimenten quantifiziert wurden. Die schwarze vertikale Linie zeigt die Grenze zwischen positiven (= höhere Expression in Synoviozyten) und negativen (= höhere Expression in Chondrozyten) LFQ-Verhältnissen an. Die gestrichelten Linien zeigen jeweils einen 2- bzw. 10-fachen Unterschied in der Proteinexpression an (entspricht ± 0,3 und ± 1 auf der logarithmischen Skala).

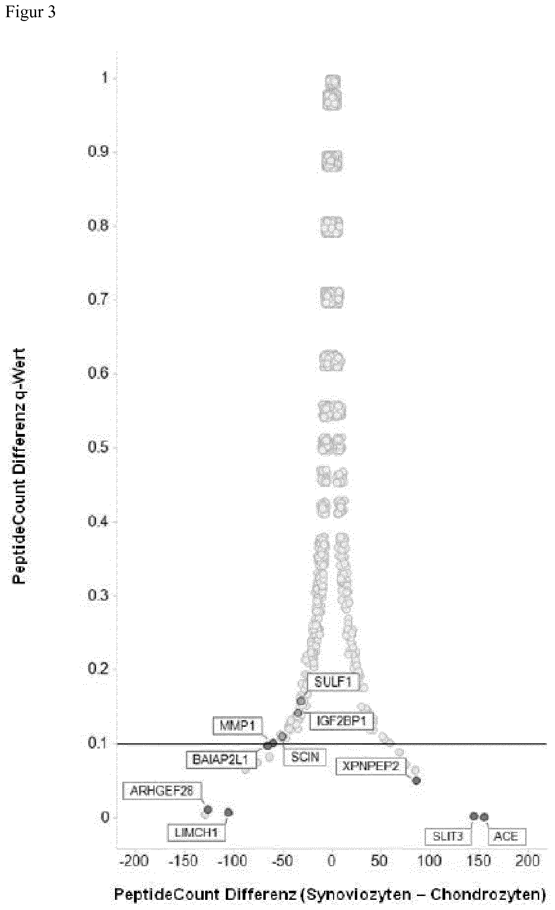

Fig. 3 zeigt ein Vulkan-Plot zur Visualisierung der Unterschiede im Peptid Count zwischen Synoviozyten und Chondrozyten und den zugehörigen Unterschieden im q-Wert für alle Proteine, die nicht im Mean Rank-Test verwendet wurden. Peptid Count-Unterschiede größer 1 bedeuten eine höhere Expression in Synoviozyten und Peptid Count-Unterschiede kleiner 1 bedeuten eine höhere Expression in Chondrozyten. Die in der Studie 1 schon als Biomarkerkandidaten eingestuften Proteine sind mittels Gennamen gekennzeichnet.

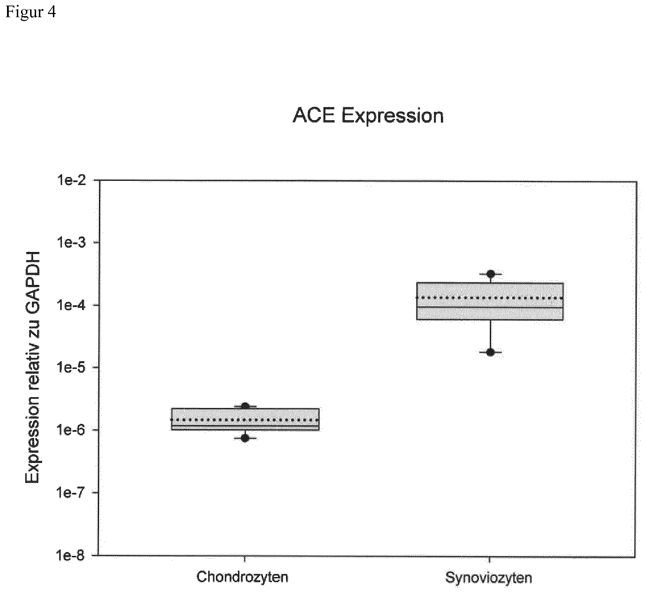

Fig. 4 zeigt graphisch die mRNA-Expression des Synoviozyten-Markers ACE. Chondrozyten und Synoviozyten desselben Donors wurden - wie nachfolgend unter 1. Material und Methoden beschrieben - kultiviert. Am Ende der Kultivierung wurde die mRNA-Expression von ACE analysiert. Die Ergebnisse sind relativ zur Expression des Referenzgenes GAPDH (Glycerinaldehyd-3-phosphat-Dehydrogenase) dargestellt (n = 10). Die in der Graphik verwendeten Boxplots zeigen die 25/75 Perzentilen (Boxen), 10/90 Perzentilen (Klammern), 5/95 Perzentilen (Punkte), die Mediane (durchgezogene Linien) und die Mittelwerte (gepunktete Linien). Die Fig. 4 veranschaulicht, dass die mRNA-Expression von ACE in Synoviozyten signifikant höher ist als in Chondrozyten. ACE ist daher in besonderer Weise zur Verwendung als Synoviozyten-Marker geeignet.

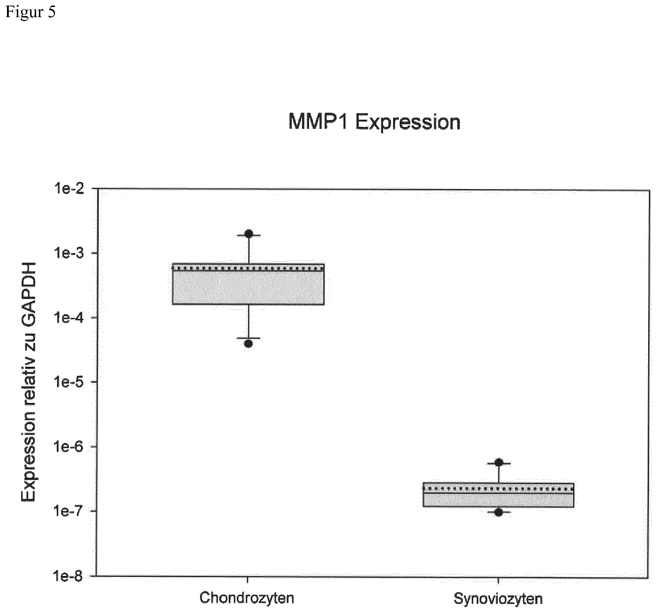

Fig. 5 zeigt graphisch die mRNA-Expression des Chondrozyten-Markers MMP1. Chondrozyten und Synoviozyten desselben Donors wurden - wie nachfolgend unter 1. Material und Methoden beschrieben - kultiviert. Am Ende der Kultivierung wurde die mRNA-Expression von MMP1 analysiert. Die Ergebnisse sind relativ zur Expression des Referenzgenes GAPDH (Glycerinaldehyd-3-phosphat-Dehydrogenase) dargestellt (n = 10). Die in der Graphik verwendeten Boxplots zeigen die 25/75 Perzentilen (Boxen), 10/90 Perzentilen (Klammern), 5/95 Perzentilen (Punkte), die Mediane (durchgezogene Linien) und die Mittelwerte (gepunktete Linien). Die Fig. 5 macht deutlich, dass die mRNA-Expression von MMP1 in Chondrozyten signifikant höher ist als in Synoviozyten. MMP1 ist daher in besonderer Weise zur Verwendung als ChondrozytenMarker geeignet.

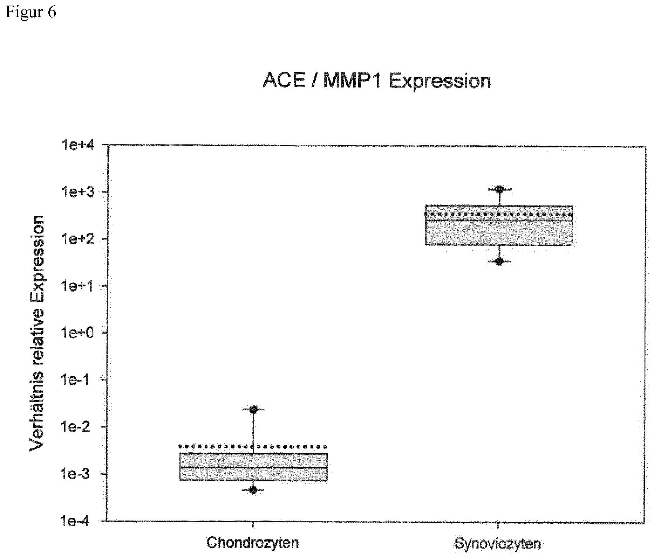

Fig. 6 zeigt graphisch das Verhältnis der Expression des Synoviozyten-Markers ACE und des Chondrozyten-Markers MMP1 in Chondrozyten und Synoviozyten (n = 10). Die in der Graphik verwendeten Boxplots zeigen die 25/75 Perzentilen (Boxen), 10/90 Perzentilen (Klammern), 5/95 Perzentilen (Punkte), die Mediane (durchgezogene Linien) und die Mittelwerte (gepunktete Linien). Fig. 6 veranschaulicht, dass sich auch das Verhältnis von ACE zu MMP1 (ACE/MMP1 Verhältnis) als Marker im Sinne der vorliegenden Erfindung, insbesondere zur Beurteilung der Zusammensetzung oder Reinheit einer Zellkultur und/oder zur in vitro Bestimmung der Identität eines Chondrozyten und/oder eines Synoviozyten eignet.

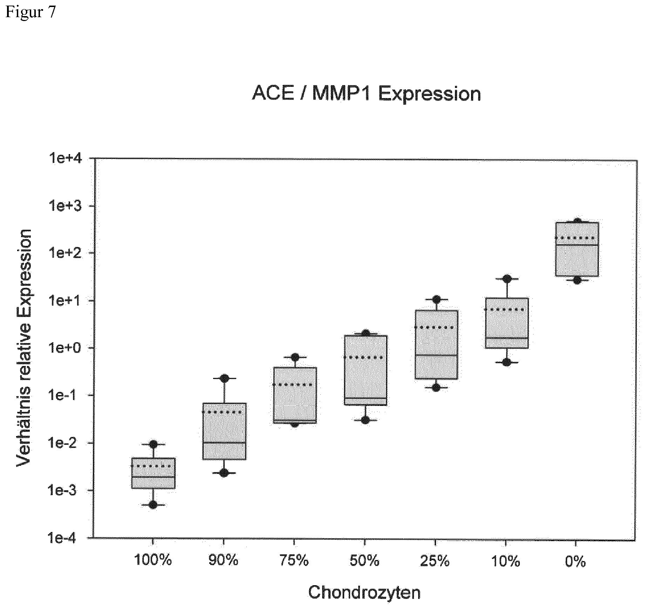

Fig. 7 zeigt graphisch das Verhältnis der mRNA-Expression von ACE zu MMP1 in Zellmischungen, welche unterschiedliche Anteile an Chondrozyten und Synoviozyten enthalten (n = 7). Die in der Graphik verwendeten Boxplots zeigen die 25/75 Perzentilen (Boxen), 10/90 Perzentilen (Klammern), 5/95 Perzentilen (Punkte), die Mediane (durchgezogene Linien) und die Mittelwerte (gepunktete Linien). Die Fig. 7 veranschaulicht, dass der Wert des Verhältnisses von ACE zu MMP1 mit dem Reinheitsgrad einer Chondrozyten-haltigen Zellmischung korreliert, und zwar in der Weise, dass ein niedriger Wert des Verhältnisses von ACE zu MMP1 mit einem hohen Reinheitsgrad einer Chondrozyten-haltigen Zellmischung und ein hoher Wert des Verhältnisses von ACE zu MMP1 mit einem niedrigen Reinheitsgrad einer Chondrozyten-haltigen Zellmischung korreliert. Fig. 7 zeigt insbesondere, dass der Reinheitsgrad einer Chondrozyten-haltigen Zellmischung umso höher ist, je niedriger der Wert des Verhältnisses von ACE zu MMP1 ist.

Die Zellmischungen wurden folgendermaßen hergestellt:
Chondrozyten und Synoviozyten wurden - wie nachfolgend unter 1. Material und Methoden beschrieben - getrennt voneinander kultiviert und am Ende der Kultur abtrypsiniert und abzentrifugiert. Das erhaltene Zellpellet wurde resuspendiert. Die Zellzahl der Zellsuspensionen wurde bestimmt und anschließend wurden die entsprechenden Volumina jeder Zellsuspension verwendet, um Mischungen mit 100%, 90%, 75%, 50%, 25%, 10% und 0% Chondrozyten herzustellen. Die so entstandenen Zellmischungen wurden erneut abzentrifugiert und das entstandene Zellpellet lysiert und für die Genexpressionsanalyse verwendet.

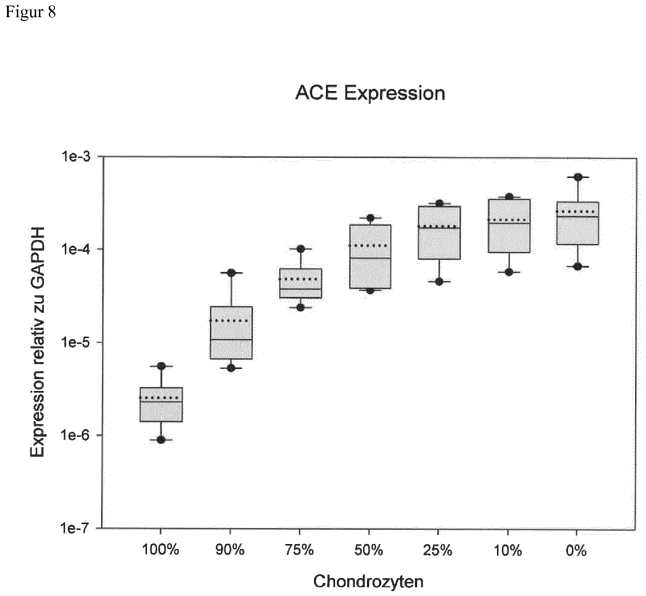

Fig. 8 zeigt graphisch die mRNA-Expression von ACE in Zellmischungen, die unterschiedliche Anteile an Chondrozyten und Synoviozyten enthalten (n = 7). Die in der Graphik verwendeten Boxplots zeigen die 25/75 Perzentilen (Boxen), 10/90 Perzentilen (Klammern), 5/95 Perzentilen (Punkte), die Mediane (durchgezogene Linien) und die Mittelwerte (gepunktete Linien).

Bezüglich der Herstellung der Zellmischungen wird auf die in Bezug auf Fig. 7 gemachten Ausführungen verwiesen.

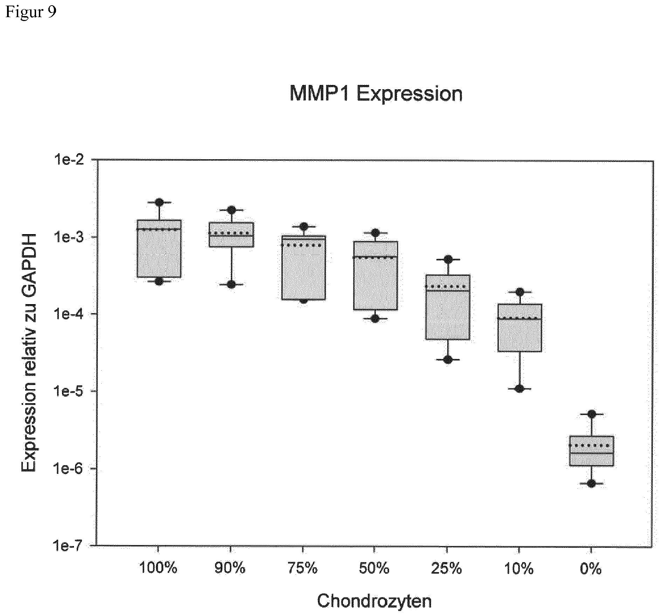

Fig. 9 zeigt graphisch die mRNA-Expression von MMP1 in Zellmischungen, die unterschiedliche Anteile an Chondrozyten und Synoviozyten enthalten (n = 7). Die in der Graphik verwendeten Boxplots zeigen die 25/75 Perzentilen (Boxen), 10/90 Perzentilen (Klammern), 5/95 Perzentilen (Punkte), die Mediane (durchgezogene Linien) und die Mittelwerte (gepunktete Linien).

Bezüglich der Herstellung der Zellmischungen wird ebenfalls auf die im Zusammenhang der Fig. 7 gemachten Ausführungen Bezug genommen.

### DETAILLIERTE BESCHREIBUNG DER AUSFÜHRUNGSBEISPIELE

### 1. Material und Methoden

### 1.1 Zellkultur, Inhibitorbehandlung und Zelllyse

Die verwendeten Chondrozyten und Synoviozyten stammten aus dem Kniegelenk verstorbener Spender aus den USA. Das Gewebe wurde mittels zweier Skalpelle sehr fein zerkleinert und mit Kollagenase für 24h, 5% CO2 und 37°C, verdaut. Das Synovialgewebe wurde während der ganzen Kultivierung identisch zum Knorpelgewebe behandelt. Nach dem Verdau erfolgte eine Inkubation in Chondrozytenmedium für 48 h, 5% CO2 und 37°C stehend. Anschließend wurden die Zellen durch einen Zellsieb gegeben, abzentrifugiert und in frischem Chondrozytenmedium expandiert. Die Zellen wurden bei 2-3 maligem Medienwechsel je nach Wachstum 19 bis maximal 23 Tage kultiviert.

Nach der Kultivierung wurden die Zellen mit Trypsin von der Kulturoberfläche abgelöst, mit PBS gewaschen, eingefroren und auf Trockeneis zu Evotec München versendet.

Die Zelllyse und die enzymatische Spaltung der Proteine wurde gemäß eines kürzlich publizierten Protokolls durchgeführt (Kulak, N.A., Pichler, G., Paron, I., Nagaraj, N., and Mann, M. (2014). Minimal, encapsulated proteomic-sample processing applied to copy-number estimation in eukaryotic cells. Nat Methods 11, 319-324). Die Zellen wurden zunächst bei -80 °C gelagert, dann bevor der Lyse auf Eis gestellt. Die Lyse wurde mit einem kalten Lyse-Puffer (1% w/v SDC, 10 mM TCEP, 40 mM CAA, 100 mM Tris pH 8,5) durchgeführt. Zellextrakte wurden sofort bei 99°C für 10 min. inkubiert und anschließend auf Eis dreimal für 30 Sekunden mit Ultraschall behandelt. Danach wurde der Zelldebris durch Zentrifugation entfernt. Der Überstand wurde in eine neues Reaktionsgefäß überführt und die Proteinkonzentration bestimmt (BCA Assay, Pierce). Für die anschließende enzymatische Proteinspaltung wurden von jeden Zelllysat 150 µg verwendet. Insgesamt wurden je 10 Chondrozyten- und 10 Synoviozytenkulturen für die Analyse verwendet.

### 1.2 Probenvorbereitung für die Massenspektrometrie (MS)

Die Proteine in den Zelllysaten wurden mit 10 MM TCEP (Tris(2-Carboxyethyl)phosphin) reduziert und in Gegenwart von 40 mM CAA (2-Chloroacetamid) alkyliert. Nach Zentrifugation wurde jeweils ein 150 µg Aliquot des klaren Lysates 1: 2 mit H2O verdünnt und eine 1 : 1 Mischung von Endopeptidase Lys-C (Wako) und Trypsin (Promega) in einem Enzym zu Protein Verhältnis von 1 : 50 zugegeben und über Nacht inkubiert. Die entstandene Peptid-Mischung wurde durch die Zugabe von 99% Ethylacetat und 1 % TFA angesäuert und anschließend mittels einer Strata-X-C Säule (100 mg Sorptionsmittel, Phenomenex) entsalzt. Die Peptide wurden mit 80 % Acetonitril und 5 % v/v Ammoniumhydroxid eluiert, in flüssigem Stickstoff schockgefroren und lyophilisiert. Anschließend wurden die Peptide mittels Reversed Phase Chromatografie bei hohem pH fraktioniert (Wang, Y., Yang, F., Gritsenko, M.A., Clauss, T., Liu, T., Shen, Y., Monroe, M.E., Lopez-Ferrer, D., Reno, T., Moore, R.J., et al. (2011). Reversed-phase chromatography with multiple fraction concatenation strategy for proteome profiling of human MCF10A cells. Proteomics 11, 2019-2026). Dazu wurden die Peptide in 20 mM Ammoniumformiat (pH 10, Puffer A) rekonstituiert, dann auf eine XBridge C18, 200 x 4,6 mm analytische Säule (Waters) eines Äkta Explorer System (GE Healthcare) geladen und mittels eines segmentierten Gradienten mit aufsteigender Acetonitril Konzentration von 7 % bis 30 % Puffer B (Puffer A mit 80 % Acetronitril) über 15 min. und anschließend für 5 min. mit einem Gradienten bis 55 % aufgetrennt. Die gesammelten Fraktionen der eluierten Peptide wurden dann in nicht-linearer Weise so vereint, dass insgesamt 12 Proben entstanden. Die Proben wurden dann mit einer selbst hergestellten C18 STAGEtip Säulen entsalzt (Rappsilber, J., Mann, M., and Ishihama, Y. (2007). Protocol for micro-purification, enrichment, pre-fractionation and storage of peptides for proteomics using StageTips. Nat Protoc 2, 1896-1906). Für die massenspektrometrische Analyse wurden die Peptide in 0,5 % Ameisensäure rekonstituiert.

### 1.3 Massenspektrometrische Analyse

Alle LC-MS/MS Analysen wurden auf einem Q Exactive Plus Massenspektrometer (Thermo Fisher Scientific) mit Easy n-LC 1000 UPLC System (Thermo Fisher Scientific) durchgeführt. Die Proben wurden mit einem Autosampler auf eine 40 cm Quarzglas-Emmissionsquelle (New Objective) geladen, die mit reverse phase Material (Reprusil-Pur C18-AQ, 3 µm, Dr. Maisch GmbH) bis zu einem maximalen Druck von 800 bar gefüllt wurde. Die gebundenen Peptide wurden in einer Laufzeit von 125 min. für die Analyse aller fraktionierten Proben eluiert. Peptide wurden mittels einer Nanoelektrospray-Ionenquelle (Proxeon Biosystems) direkt in das Massenspektrometer gesprüht. Das Massenspektrometer wurde in einem Daten abhängigen Modus betrieben, um automatisch zwischen MS Gesamtscans in einer Auflösung von R = 70.000 (bei m/z = 200) mit einem Targetwert von 3.000.000 Counts (max. Injektionszeit = 45 ms) und MS/MS Fragmentationsscans bei R = 17.500 und einem Targetwert von 100.000 Ionen wechseln zu können. Die 10 intensivsten Peptid-Ionen wurden mittels HCD (higher-energy collisional dissociation) ausgewählt.

### 1.4 Datenverarbeitung

Alle Rohdaten, die im Rahmen der Untersuchungen erhalten wurden, wurden mit der MaxQuant Software Suite (Version 1.5.3.2) für Peptid und Protein-Identifizierung und Quantifizierung unter Nutzung der humanen SwissProt Datenbank (Version 03 2014) prozessiert (Cox, J., and Mann, M. (2008). MaxQuant enables high peptide identification rates, individualized p.p.b.-range mass accuracies and proteome-wide protein quantification. Nat Biotechnol 26, 1367-1372; Olsen, J.V., Vermeulen, M., Santamaria, A., Kumar, C., Miller, M.L., Jensen, L.J., Gnad, F., Cox, J., Jensen, T.S., Nigg, E.A., et al. (2010). Quantitative phosphoproteomics reveals widespread full phosphorylation site occupancy during mitosis. Sci Signal 3, ra3). Die markierungsfreie Quantifizierungsmöglichkeit wurde aktiviert, während die "match between runs" Funktion abgeschaltet wurde. Die maximal zulässige Abweichung der Massen war 4,5 ppm für MS und 20 ppm für MS2 Peaks. Carbamidomethylierung von Cysteinen wurde als feste Modifikation eingegebenen, Oxidation von Methionin und N-terminale Acetylierung wurden als variable Modifikationen festgelegt. Alle Peptide mussten eine minimale Länge von 7 Aminosäuren aufweisen und es wurde ein Maximum von zwei nicht gespaltenen tryptischen Schnittstellen (missed cleavages) zugelassen. Die FDR (false discovery rate) für Protein- und Peptid-Identifizierung wurde auf 1 % gesetzt.

### 1.5 Bioinformatische Datenanalyse

Die Proteinintensitäten der markierungsfreien Quantifizierung (LFQ, label-free quantification) wurden für die Kalkulation der Verhältnis der Synoviozyten und der Chondrozyten desselben Donors verwendet. Dieses Paaren resultierte in insgesamt 5 Replikaten. Die erhaltenen Verhältnisse wurden dann log10-transformiert und für die weitere Datenanalyse verwendet. Proteine, die als Kontaminanten oder reverse-hits in der Decoy-Datenbank markiert waren, wurden aus der modifizierten MaxQuant Proteingruppen-Tabelle entfernt. Signifikante Expressionsunterschiede von Proteinen mit LFQ-Verhältnissen in drei oder mehr Replikaten wurden mittels dem nicht-parametrischen "one-sample MeanRank"-Test (Klammer M., Dybowski J.N., Hoffmann D., and Schaab C. (2014). Identification of significant features by the Global Mean Rank test. PLoS One. 2014, Aug 13;9(8):e104504. doi: 10.1371) bestimmt. Der MeanRank-Test ist ein globaler Rang-basierter Test, der die FDR (false discovery rate) zuverlässig kontrolliert.

Proteine mit Verhältnissen in weniger als 3 Replikaten wurden einem nicht-parametrischen "PeptideCount"-Test unterzogen, der die individuellen Peptidcounts für ein gegebenes Protein benützt. Für jedes Protein wurde der mittlere Unterschied in den PeptideCounts zwischen Chondrozyten und Synoviozyten bestimmt. Gruppen-Labels wurden dann permutiert und das Auftreten von größeren Unterschieden wurde über alle Proteine hinweg gezählt. Das wurde für alle möglichen Permutationen wiederholt, indem die durchschnittliche Zahl von falsch entdeckten benutzt wurde, um die false discovery (FD) eines gegebenen Proteins abzuschätzen.

Für die kombinierte Analyse der beiden Studien wurden insgesamt 10 Donoren mit zusammengehörigen Chondrozyten und Synoviozyten verwendet. Signifikante Expressionsunterschiede in Proteinen mit LFQ Verhältnissen in mindestens 7 Replikaten wurden mittels MeanRank-Test bestimmt und alle anderen Proteine wurden dem nicht-parametrischen "PeptideCount"-Test unterworfen.

### 1.6 Vergleich der beiden Datensätze:

Um die Ergebnisse der beiden Einzelstudien mit je 5 Donoren vergleichen zu können, wurden die Daten entweder über die UniProtKB Identifier und/oder über den Genname abgeglichen. Basierend auf der Qualität des Abgleiches wurden diese in die folgenden vier Kategorien eingeteilt: (++) identische UniProtKB Identifier; (+) teilweise identische UniProtKB Identifier und identische Gennamen; (+/-) teilweise identische UniProtKB Identifier und teilweise passender Genname; (-) nichts zutreffendes.

### 1.7 Genexpressionsanalyse

Am Ende der Kultivierung wurde von den abgelösten Zellen ein Aliquot von 0,5 Mio. für die Analyse der Genexpression abgenommen, abzentrifugiert und das Zellpellet mit Lysepuffer lysiert. Die anschließende RNA Isolation erfolgt mittels RNeasy mini kit 250 (Qiagen). Die cDNA Synthese erfolgt mittels Advantage^{®} RT for PCR kit (Clontech).

Genspezifische Primersequenzen wurden am NCBI mit PrimerBlast designed und von Biotez synthetisiert. Die Primer wurden in einer Konzentration von 100nM bzw. 200nM/Well verwendet und die Expression der mRNA mittels des real-time PCR Verfahren am LC480 von Roche analysiert. Zur Detektion der amplifizierten Sequenzen wurde der Fluoreszenzfarbstoff SYBR Green verwendet.

### 2. Ergebnisse:

Insgesamt konnten bei einer festgelegten FDR von < 1 % 10.702 verschiedene Proteine identifiziert werden. Für Chondrozten und Synoviozyten wurde eine ähnliche Anzahl von Proteinen identifiziert (10.550 bzw. 10.462). Die Identifizierungen basieren auf mehr als 2.300.000 Peptid-Evidenzen und auf 162.299 identifizierten Peptiden. Über alle Experimente hinweg konnte eine vergleichbare Anzahl von Proteinen identifiziert werden, was für die grundsätzliche Robustheit des angewendeten Workflows für die Proteomanalyse spricht.

Um die Reproduzierbarkeit der Proteinquantifizierung über biologische Replikate von 5 Donoren mit zusammengehörigen Chondrozyten- und Synoviozyten-Präparationen hinweg zu zeigen, wurden Scatterplot-Analysen der markierungsfreien Quantifizierungsverhältnisse (LFQ ratio) durchgeführt. Dazu wurden die Intensitäten der LFQ in log10-transformierte Verhältnisse umgewandelt, die das Verhältnis der Synoviozyten LFQ-Intensität zur LFQ-Intensität der Chondrozyten darstellen.

Exemplarisch sind die Ergebnisse für Donor 1 in Figur 1 dargestellt. Neben einer großen Wolke von log10-transformierten Proteinverhältnissen um Null herum (= um ein lineares Verhältnis von etwa 1 herum), was bedeutet, dass die Proteine in beiden Zelltypen gleich exprimiert sind, findet man einige Proteine, die eine unterschiedliche Häufigkeit in den beiden Zelltypen besitzen. Die Scatterplot-Vergleiche zeigen eine hohe Korrelation der Donoren, da die überwiegende Mehrheit der Proteine auf einer Ursprungsgerade mit der Steigung 1 liegen. Die erhaltenen Ergebnisse waren für beide Studien vergleichbar.

Im nächsten Schritt wurden weitere Datenanalysen durchgeführt, um Proteine mit signifikant unterschiedlichen Expressionsniveaus zwischen den beiden Zelltypen zu identifizieren. Dafür wurden alle Proteine, die in mindestens 3 der 5 biologischen Replikaten quantifiziert wurden, einer statistischen Analyse unterworfen. Insgesamt erfüllten 7.361 Proteine in Studie 2 und 5.378 Proteine in Studie 1 diese Anforderungen. Signifikante und reproduzierbare Veränderungen wurden durch Anwendung des MeanRank-Test bei einer FDR von 10 % (q-Werte ≤ 0,1) (Klammer M., Dybowski J.N., Hoffmann D., and Schaab C. (2014). Identification of significant features by the Global Mean Rank test. PLoS One. 2014, Aug 13;9(8):e104504. doi: 10.1371) identifiziert. Die angewandte FDR von 10 % ist dabei ein willkürlich gewählter Wert, der als geeignet für die weiteren bioinformatischen Analysen der Studie, wie gene ontology enrichment und interaction-network analysis, erschien. Die Verteilung der Proteine mit signifikant unterschiedlicher Proteinexpression zwischen den beiden Zelltypen kann mit Hilfe von sogenannten "Vulkanplots" visualisiert werden. Hierfür wurden die Mittelwerte der log10-transformierten Synoviozyten/Chondrozyten LFQ Intensitäten (mean LFQ ratio) gegen die Standardabweichung der Verhältnisse aller Proteine aufgetragen. Alle Proteine mit differentieller Expression zwischen den beiden Zelltypen sind in rot dargestellt. Proteine, die als "Biomarker-Kandidaten" eingestuft wurden, wurden namentlich markiert.

Nach Anwendung des MeanRank-Test erwiesen sich ca. 15 % aller Proteine, die im Test analysiert wurden, als signifikant unterschiedlich in Chondrozyten und Synoviozyten (insgesamt 1.135). Um Markerproteine zu identifizieren, die möglichst exklusiv nur in einem der beiden Zelltypen exprimiert wurden, wurde im nächsten Schritt ein "PeptideCount" durchgeführt. Dieser Test vergleicht die durchschnittliche Anzahl von Peptiden, mit denen ein Protein in den beiden Zelltypen identifiziert wurde. Insgesamt wurden für diesen Test 3.340 Proteine verwendet, von denen 16 Proteine differentiell exprimiert waren (10 % FDR, q-Werte ≤ 0,1). Von diesen 16 Proteinen waren 8 jeweils nur in einem Zelltyp exprimiert bzw. in einem Zelltyp signifikant höher exprimiert.

Ausgewählte Proteine mit spezifischer Proteinexpression in Chondroyzten oder Synoviozyten, die anhand des "PeptideCount"-Test in beiden Studien gefunden wurden, sind in Tabelle 1 dargestellt.

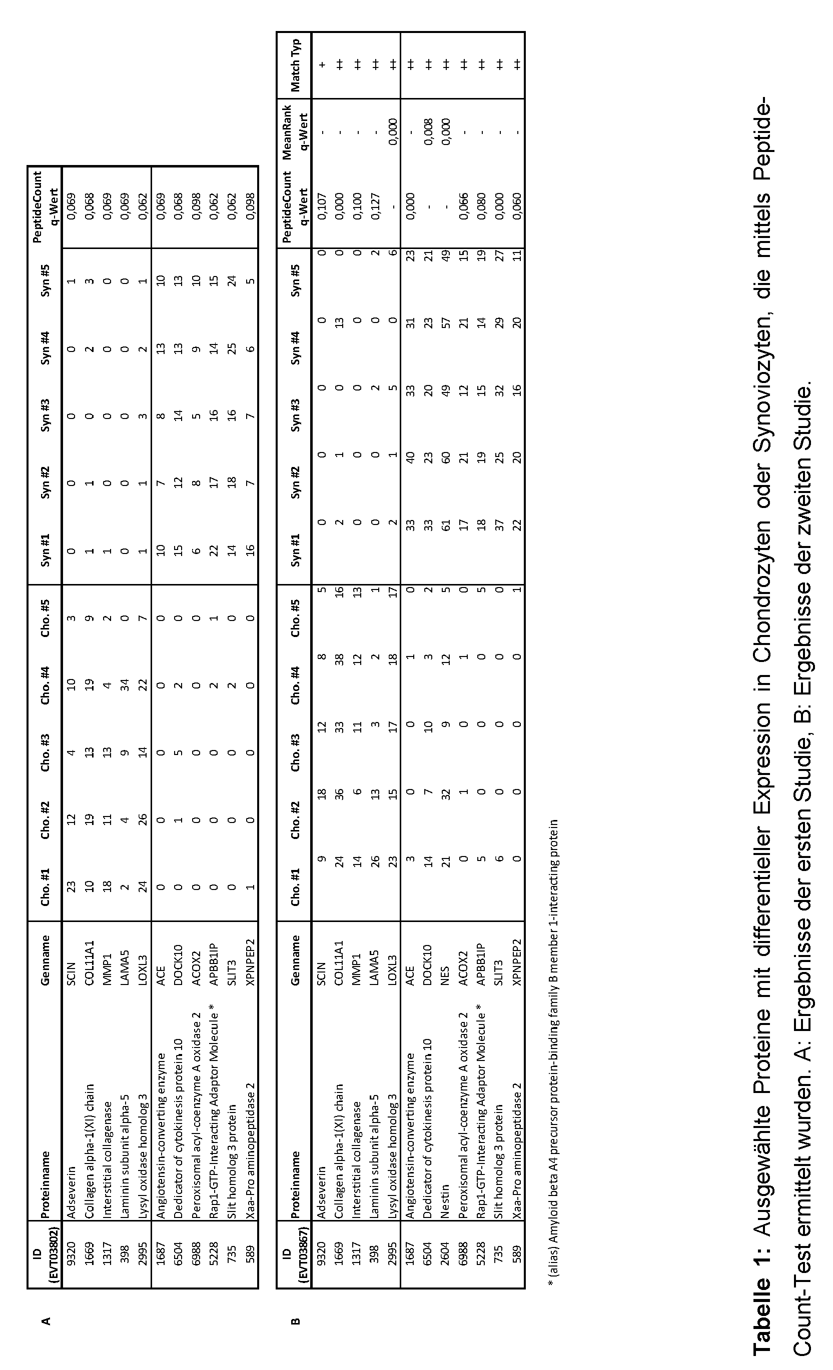

Proteine, die mittels bioinformatischer Analyse als signifikant unterschiedlich exprimiert ermittelt wurden, sind in Tabelle 2 zusammengestellt.

**Tabelle 2: Marker für Chondrozyten und Synoviozyten.**

| **Zelltyp** | **ID (Proteingruppe, EVT03802)** | **ID (Proteingruppe, EVT03867)** | **Proteinname** | **Genname** | **Match Typ** | **angew. Test (EVT03802)** | **q-Wert (EVT03802)** | **angew. Test (EVT03867)** | **q-Wert (EVT03867)** |
|---|---|---|---|---|---|---|---|---|---|
| **Chondrozyten** | 4057 | 4524 | Periostin | POSTN | ++ | MeanRank | 0,009 | MeanRank | 0,009 |
| | 5679 | 6480 | Synaptopodin | SYNPO | + | PeptideCount | 0,068 | MeanRank | 0,023 |
| | 1317 | 1522 | Interstitial collagenase | MMP1 | ++ | PeptideCount | 0,069 | PeptideCount | 0,100 |
| | 5512 | 6273 | Extracellular sulfatase Sulf-1 | SULF1 | ++ | MeanRank | 0,009 | PeptideCount | 0,162 |
| | 5645 | 6432 | Rho guanine nucleotide exchange factor 28 | ARHGEF28 | + | PeptideCount | 0,098 | PeptideCount | 0,003 |
| | 8360 | 9665 | Insulin-like growth factor 2 mRNA-binding protein 1 | IGF2BP1 | + | PeptideCount | 0,098 | PeptideCount | 0,134 |
| | 8638 | 9976 | Brain-specific angiogenesis inhibitor 1-associated protein 2-like protein 1 | BAIAP2L1 | ++ | PeptideCount | 0,098 | PeptideCount | 0,091 |
| | 8901 | 10270 | LIM and calponin homology domains-containing protein 1 | LIMCH1 | + | PeptideCount | 0,068 | PeptideCount | 0,014 |
| | 9320 | 10748 | Adseverin | SCIN | + | PeptideCount | 0,069 | PeptideCount | 0,107 |
| **Synoviozyten** | 367 | 440 | Serine/threonine-protein kinase DCLK1 | DCLK1 | + | MeanRank | 0,000 | MeanRank | 0,000 |
| | 640 | 763 | Periplakin | PPL | + | MeanRank | 0,000 | MeanRank | 0,004 |
| | 2164 | 2455 | Cellular retinoic acid-binding protein 2 | CRABP2 | ++ | MeanRank | 0,000 | MeanRank | 0,000 |
| | 2604 | 2927 | Nestin | NES | ++ | PeptideCount | 0,000 | MeanRank | 0,000 |
| | 589 | 703 | Xaa-Pro aminopeptidase 2 | XPNPEP2 | ++ | PeptideCount | 0,098 | PeptideCount | 0,060 |
| | 735 | 876 | Slit homolog 3 protein | SLIT3 | ++ | PeptideCount | 0,062 | PeptideCount | 0,000 |
| | 1687 | 1928 | Angiotensin-converting enzyme | ACE | ++ | PeptideCount | 0,069 | PeptideCount | 0,000 |

Für die als "Biomarker-Kandidaten" eingestuften Proteine wurde im Folgenden zusätzlich der Unterschied in der Proteinexpression zwischen den beiden Zelltypen (fold-difference) und die mittleren PeptideCounts zusammengefasst (Tabelle 3).

**Tabelle 3: Unterschiede in der Expression der Marker zwischen Chondroyzten und Synoviozyten sowie die Mittelwerte der PeptideCounts (jeweils getrennt nach Studie 1 und 2 aufgelistet).**

| **Zelltyp** | **Genname** | **angew. Test (EVT03802)** | **angew. Test (EVT03867)** | **EVT03802_fach Unterschied in Protein-expression** | **EVT03867_fach Unterschied in Protein-expression** | **EVT03802_Mittel wert PeptideCount Synoviozyten (#1-#5)** | **EVT03802_Mittel wert PeptideCount Chondrozyten (#1-#5)** | **EVT03867_Mittel wert PeptideCount Synoviozyten (#1-#5)** | **EVT03867_Mittel wert PeptideCount Chondrozyten (#1-#5)** |
|---|---|---|---|---|---|---|---|---|---|
| | POSTN | MeanRank | MeanRank | 46,7 | 15,2 | 7 | 54,6 | 12,4 | 30,6 |
| | SYNPO | PeptideCount | MeanRank | 17,3 | 6,0 | 0,6 | 13,2 | 4,6 | 17 |
| | MMP1 | PeptideCount | PeptideCount | | | 0,2 | 9,6 | 0 | 11,2 |
| | SULF1 | MeanRank | PeptideCount | 46,1 | 2,8 | 1,8 | 17,8 | 3,2 | 9,2 |
| **Chondrozyten** | ARHGEF28 | PeptideCount | PeptideCount | | | 0 | 7,8 | 0,8 | 25,4 |
| | IGF2BP1 | PeptideCount | PeptideCount | | | 0 | 7,6 | 0,6 | 7,8 |
| | BAIAP2L1 | PeptideCount | PeptideCount | 3,7 | 7,5 | 0,6 | 8,4 | 1,4 | 14,2 |
| | LIMCH1 | PeptideCount | PeptideCount | | 6,7 | 0,2 | 12,8 | 1,2 | 22,6 |
| | SCIN | PeptideCount | PeptideCount | | | 0,2 | 10,4 | 0 | 10,4 |
| | DCLK1 | MeanRank | MeanRank | 67,7 | 19,0 | 29,8 | 2,6 | 23,2 | 10,6 |
| | PPL | MeanRank | MeanRank | 116,5 | 4,7 | 131,6 | 10,8 | 89,6 | 56,6 |
| | CRABP2 | MeanRank | MeanRank | 71,7 | 269,8 | 14,8 | 2,8 | 8 | 2 |
| **Synoviozyten** | NES | PeptideCount | MeanRank | 26,6 | 114,3 | 60,6 | 5,8 | 55,2 | 15,8 |
| | XPNPEP2 | PeptideCount | PeptideCount | | | 8,2 | 0,2 | 17,8 | 0,2 |
| | SLIT3 | PeptideCount | PeptideCount | 23,2 | 81,4 | 19,4 | 0,4 | 30 | 1,2 |
| | ACE | PeptideCount | PeptideCount | | 207,7 | 9,6 | 0 | 32 | 0,8 |

## Patentansprüche

1. Verwendung eines Markers zur Beurteilung der Zusammensetzung oder Reinheit einer Zellkultur, wobei es sich bei dem Marker um wenigstens einen Marker handelt, welcher aus der Gruppe bestehend aus ACE, MMP1, POSTN, SYNPO, SULF1, ARHGEF28, IGF2BP1, BAIAP2L1, LIMCH1, SCIN, DCLK1, PPL, CRABP2, NES, XPNPEP2 und SLIT3 ausgewählt ist, wobei das Expressionsniveau des wenigstens einen Markers bestimmt wird, **dadurch gekennzeichnet, dass** es sich bei dem wenigstens einen Marker um ACE oder um eine Kombination von ACE und wenigstens einem weiteren Marker, ausgewählt aus der Gruppe bestehend aus MMP1, POSTN, SYNPO, SULF1, ARHGEF28, IGF2BP1, BAIAP2L1, LIMCH1, SCIN, DCLK1, PPL, CRABP2, NES, XPNPEP2 und SLIT3, und bei der Zellkultur um eine Knorpelzellkultur handelt.

2. Verwendung nach Anspruch 1, **dadurch gekennzeichnet, dass** das Expressionsniveau des wenigstens einen Markers in einer aus der Zellkultur gewonnenen Zellprobe bestimmt wird.

3. Verwendung nach Anspruch 1 oder Anspruch 2, **dadurch gekennzeichnet, dass** die Zellkultur Zellen aufweist, welche aus einem Knorpelbiopsat, bevorzugt Knorpel-Knochen-Stanzbiopsat, insbesondere einem Knorpelgewebefragment davon, stammen.

4. Verwendung nach Anspruch 3, **dadurch gekennzeichnet, dass** es sich bei dem Biopsat um ein Biopsat aus einem Gelenk, insbesondere Kniegelenk, handelt.

5. Verwendung nach einem der vorhergehenden Ansprüche, **dadurch gekennzeichnet, dass** es sich bei dem wenigstens einen Marker um das Verhältnis von ACE zu MMP1 oder um das umgekehrte Verhältnis davon handelt.

6. Verwendung nach Anspruch 5, **dadurch gekennzeichnet, dass** der Wert des Verhältnisses von ACE zu MMP1 oberhalb eines definierten Schwellenwertes anzeigt, dass die Zellkultur Synovialzellen aufweist, wobei der Schwellenwert vorzugsweise gleich oder kleiner ist als der Wert des Verhältnisses von ACE zu MMP1 in einer reinen Synovialzellkultur.

7. Verwendung nach einem der vorhergehenden Ansprüche, **dadurch gekennzeichnet, dass** das Expressionsniveau auf RNA-Basis, vorzugsweise mRNA-Basis, oder auf Protein-Basis bestimmt wird.

8. Verfahren zur Beurteilung der Zusammensetzung oder Reinheit einer Zellkultur, aufweisend die folgenden Schritte:
a) Gewinnen einer Zellprobe aus einer Zellkultur,
b) Bestimmen des Expressionsniveaus wenigstens eines Markers in der Zellprobe und
c) Bestimmen der Zusammensetzung oder Reinheit der Zellkultur basierend auf dem Expressionsniveau des wenigstens einen Markers,
**dadurch gekennzeichnet, dass** der wenigstens eine Marker ACE oder eine Kombination von ACE und wenigstens einem weiteren Marker, ausgewählt aus der Gruppe bestehend aus MMP1, POSTN, SYNPO, SULF1, ARHGEF28, IGF2BP1, BAIAP2L1, LIMCH1, SCIN, DCLK1, PPL, CRABP2, NES, XPNPEP2 und SLIT3, ist, und es sich bei der Zellkultur um eine Knorpelzellkultur handelt.

9. Verwendung eines Kits zur Durchführung eines Verfahrens nach Anspruch 8, wobei das Kit wenigstens eine Komponente zum Nachweis wenigstens eines Markers, ausgewählt aus der Gruppe bestehend aus MMP1, ACE, POSTN, SYNPO, SULF1, ARHGEF28, IGF2BP1, BAIAP2L1, LIMCH1, SCIN, DCLK1, PPL, CRABP2, NES, XPNPEP2 und SLIT3, aufweist, **dadurch gekennzeichnet, dass** es sich bei dem wenigstens einen Marker um ACE oder um eine Kombination von ACE und wenigstens einem weiteren Marker, ausgewählt aus der Gruppe bestehend aus MMP1, POSTN, SYNPO, SULF1, ARHGEF28, IGF2BP1, BAIAP2L1, LIMCH1, SCIN, DCLK1, PPL, CRABP2, NES, XPNPEP2 und SLIT3 handelt.

## Claims

1. Use of a marker for assessing the composition or purity of a cell culture, wherein the marker is at least one marker selected from the group consisting of ACE, MMP1, POSTN, SYNPO, SULF1, ARHGEF28, IGF2BP1, BAIAP2L1, LIMCH1, SCIN, DCLK1, PPL, CRABP2, NES, XPNPEP2 and SLIT3, wherein the expression level of the at least one marker is determined, **characterized in that** the at least one marker is ACE or a combination of ACE and at least one further marker selected from the group consisting of MMP1, POSTN, SYNPO, SULF1, ARHGEF28, IGF2BP1, BAIAP2L1, LIMCH1, SCIN, DCLK1, PPL, CRABP2, NES, XPNPEP2 and SLIT3, and the cell culture is a chondrocyte culture.

2. Use according to claim 1, **characterized in that** the expression level of the at least one marker is determined in a cell sample obtained from the cell culture.

3. Use according to claim 1 or claim 2, **characterized in that** the cell culture contains cells that originate from a cartilage biopsy specimen, preferably a cartilage/bone punch biopsy specimen, more particularly a cartilage tissue fragment thereof.

4. Use according to claim 3, **characterized in that** the biopsy specimen is a biopsy specimen from a joint, more particularly a knee joint.

5. Use according to any of the preceding claims, **characterized in that** the at least one marker is the ratio of ACE to MMP1 or the inverse ratio of the two.

6. Use according to claim 5, **characterized in that** the value of the ratio of ACE to MMP1 above a defined threshold level indicates that the cell culture contains synovial cells, wherein the threshold value is preferably equal to or less than the value of the ratio of ACE to MMP1 in a pure synovial cell culture.

7. Use according to any of the preceding claims, **characterized in that** the expression level is determined on an RNA basis, preferably an mRNA basis, or a protein basis.

8. Method for assessing the composition or purity of a cell culture, comprising the following steps:
a) obtaining a cell sample from a cell culture,
b) determining the expression level of at least one marker in the cell sample, and
c) determining the composition or purity of the cell culture based on the expression level of the at least one marker,
**characterized in that** the at least one marker is ACE or a combination of ACE and at least one further marker selected from the group consisting of MMP1, POSTN, SYNPO, SULF1, ARHGEF28, IGF2BP1, BAIAP2L1, LIMCH1, SCIN, DCLK1, PPL, CRABP2, NES, XPNPEP2 and SLIT3, and the cell culture is a chondrocyte culture.

9. Use of a kit for carrying out a method according to claim 8, wherein the kit comprises at least one component for detection of at least one marker selected from the group consisting of MMP1, ACE, POSTN, SYNPO, SULF1, ARHGEF28, IGF2BP1, BAIAP2L1, LIMCH1, SCIN, DCLK1, PPL, CRABP2, NES, XPNPEP2 and SLIT3, **characterized in that** the at least one marker is ACE or a combination of ACE and at least one further marker selected from the group consisting of MMP1, POSTN, SYNPO, SULF1, ARHGEF28, IGF2BP1, BAIAP2L1, LIMCH1, SCIN, DCLK1, PPL, CRABP2, NES, XPNPEP2 and SLIT3.

## Revendications

1. Utilisation d'un marqueur pour l'évaluation de la composition ou de la pureté d'une culture cellulaire, le marqueur étant au moins un marqueur choisi dans le groupe constitué par ACE, MMP1, POSTN, SYNPO, SULF1, ARHGEF28, IGF2BP1, BAIAP2L1, LIMCH1, SCIN, DCLK1, PPL, CRABP2, NES, XPNPEP2 et SLIT3, le niveau d'expression de l'au moins un marqueur étant déterminé, **caractérisée en ce que** l'au moins un marqueur est ACE ou une combinaison d'ACE et d'au moins un autre marqueur, choisi dans le groupe constitué par MMP1, POSTN, SYNPO, SULF1, ARHGEF28, IGF2BP1, BAIAP2L1, LIMCH1, SCIN, DCLK1, PPL, CRABP2, NES, XPNPEP2 et SLIT3, et la culture cellulaire est une culture de cellules de cartilage.

2. Utilisation selon la revendication 1, **caractérisée en ce que** le niveau d'expression de l'au moins un marqueur est déterminé dans un échantillon cellulaire obtenu de la culture cellulaire.

3. Utilisation selon la revendication 1 ou la revendication 2, **caractérisée en ce que** la culture cellulaire présente des cellules qui proviennent d'un prélèvement biopsique de cartilage, préférablement d'un prélèvement biopsique de cartilage-poinçon à os, en particulier d'un fragment de tissu de cartilage de celui-ci.

4. Utilisation selon la revendication 3, **caractérisée en ce que** le prélèvement biopsique est un prélèvement biopsique d'une articulation, en particulier d'une articulation du genou.

5. Utilisation selon l'une quelconque des revendications précédentes, **caractérisée en ce que** l'au moins un marqueur est le rapport d'ACE sur MMP1 ou le rapport inverse.

6. Utilisation selon la revendication 5, **caractérisée en ce que** la valeur du rapport d'ACE sur MMP1 au-dessus d'une valeur seuil définie indique que la culture cellulaire présente des cellules synoviales, la valeur seuil étant de préférence égale ou inférieure à la valeur du rapport d'ACE sur MMP1 dans une culture de cellules synoviales pure.

7. Utilisation selon l'une quelconque des revendications précédentes, **caractérisée en ce que** le niveau d'expression est déterminé sur une base d'ARN, de préférence une base d'ARNm ou sur une base de protéines.

8. Procédé d'évaluation de la composition ou de la pureté d'une culture cellulaire, présentant les étapes suivantes :
a) obtention d'un échantillon cellulaire d'une culture cellulaire,
b) détermination du niveau d'expression d'au moins un marqueur dans l'échantillon cellulaire et
c) détermination de la composition ou de la pureté de la culture cellulaire sur la base du niveau d'expression de l'au moins un marqueur,
**caractérisé en ce que** l'au moins un marqueur est ACE ou une combinaison d'ACE et d'au moins un autre marqueur choisi dans le groupe constitué par MMP1, POSTN, SYNPO, SULF1, ARHGEF28, IGF2BP1, BAIAP2L1, LIMCH1, SCIN, DCLK1, PPL, CRABP2, NES, XPNPEP2 et SLIT3, et la culture cellulaire est une culture de cellules de cartilage.

9. Utilisation d'un kit pour la réalisation d'un procédé selon la revendication 8, le kit comprenant au moins un composant pour la détection d'au moins un marqueur choisi dans le groupe constitué par MMP1, ACE, POSTN, SYNPO, SULF1, ARHGEF28, IGF2BP1, BAIAP2L1, LIMCH1, SCIN, DCLK1, PPL, CRABP2, NES, XPNPEP2 et SLIT3, **caractérisée en ce que** l'au moins un marqueur est ACE ou une combinaison d'ACE et d'au moins un autre marqueur choisi dans le groupe constitué par MMP1, POSTN, SYNPO, SULF1, ARHGEF28, IGF2BP1, BAIAP2L1, LIMCH1, SCIN, DCLK1, PPL, CRABP2, NES, XPNPEP2 et SLIT3.
